# EUROPÄISCHE PATENTSCHRIFT

(11) **EP 1 882 455 B1**
(45) Veröffentlichungstag und Bekanntmachung des Hinweises auf die Patenterteilung: **18.03.2009**
(21) Anmeldenummer: 07014256.7
(22) Anmeldetag: 20.07.2007
(51) Int. Cl.: A61B 17/32, A61M 1/00, A61B 17/16

(54) **Medizinisches Instrument zum Schneiden von Gewebe**
Medical instrument for cutting tissue
Instrument médical destiné à couper des tissus

(30) Priorität: 24.07.2006 DE 102006034756
(43) Veröffentlichungstag der Anmeldung: 30.01.2008
(73) Patentinhaber: Karl Storz GmbH & Co. KG, 78532 Tuttlingen (DE)
(72) Erfinder: Berberich, Sascha, 78532 Tuttlingen (DE)
(74) Vertreter: Weller, Wolfgang

(56) Entgegenhaltungen:
- WO-A-20/06011119
- US-A- 4 811 734
- US-A- 5 741 287
- US-B1- 6 610 059

## Beschreibung

Die Erfindung betrifft ein medizinisches Instrument zum Schneiden von Gewebe, mit einem rohrförmigen Außenschaft, der im Bereich seines distalen Endes zumindest ein Fenster mit zumindest einer Schneide aufweist, und mit einem rohrförmigen, um eine Längsachse rotierbaren, in dem Außenschaft aufgenommenen Innenschaft, der an seinem distalen Ende ein im Bereich des zumindest einen Fensters des Außenschafts angeordnetes, mit mehreren umfänglich angeordneten Öffnungen versehenes Schneidelement aufweist, wobei jede der Öffnungen des Schneidelements zumindest eine Schneide aufweist, die bei dem in Rotation versetzten Schneidelement mit der zumindest einen Schneide des Außenschafts schneidend zusammenwirkt.

Ein derartiges Instrument ist aus der US 5,489,291 bekannt.

Dokument US 5,741,287 (Basis für den Oberbegriff des Anspruchs 1) offenbart auch ein derartiges Instrument.

Solche Instrumente werden in der minimal-invasiven Chirurgie zum Abtrennen von Gewebe im menschlichen oder tierischen Körper verwendet. Dazu wird das distale Ende des Schaftes zu dem Operationsgebiet geführt, in dem sich das abzutrennende Gewebe befindet. Zum Abtrennen des Gewebes wird das Schneidelement mittels eines externen oder internen Motors in Rotation versetzt. Die an dem Schneidelement ausgebildete Schneide wirkt während des Umlaufens mit einem als Schneide ausgebildeten Rand des Fensters des Schaftes schneidend zusammen, indem die Schneide des Schneidelements an der Schneide des Fensters bei jedem Umlauf vorbeiläuft. Um das abzutrennende Gewebe zwischen die zusammenwirkenden Schneiden zu bringen, ist bei solchen Instrumenten der Schaft mit einer Saugquelle verbunden, deren Saugwirkung durch den Schaft bis zu dem Fenster reicht, um das abzutrennende Gewebe durch das Fenster in den Schaft zu saugen, damit die Schneiden das Gewebe abtrennen können. Durch den Unterdruck wird das abgetrennte Gewebe durch den Schaft abgesaugt.

Das aus der eingangs genannten US 5,489,291 bekannte Instrument weist einen Außenschaft auf, der an seinem distalen Ende abgeschrägt ist. In dem Außenschaft ist ein rohrförmiger rotierbarer Innenschaft aufgenommen, an dessen distalem Ende ein Schneidelement ausgebildet ist. Das Schneidelement weist mehrere Öffnungen auf, die, in einer Umfangsrichtung gesehen, gleich breit sind. Jede der Öffnungen weist eine Schneide auf.

Das abzutrennende Gewebe wird in eine der Öffnungen des in dem Außenschaft rotierenden Schneidelements gesaugt. Das Gewebe wird danach abgetrennt, indem die als Schneide ausgebildete Kante der Öffnung des Schneidelements, in der das abzutrennende Gewebe gesaugt ist, an der in Umlaufrichtung vorderen Kante des zugespitzten Abschnittes des Außenschafts vorbeiläuft. Nachdem das Gewebe abgetrennt ist, wird es durch den Innenschaft zum proximalen Ende des Instruments hin abgesaugt.

Dadurch, dass die Breite der mehreren Öffnungen des Schneidelements, in einer Umfangsrichtung gesehen, gleich ist, stellt man fest, dass nur Gewebe, dessen Breite etwa der Breite der Öffnungen des Schneidelements entspricht, effektiv und effizient in die Öffnungen gesaugt werden kann und somit abgetrennt werden kann. Gewebe, das deutlich größer ist als die Öffnungen des Schneidelements, wird entweder gar nicht oder nur teilweise in die Öffnungen des Schneidelements gesaugt. Man hat festgestellt, dass für ein Schneidelement einer bestimmten Größe eine ebenso bestimmte Größe an Gewebe gibt, die effektiv abgetrennt werden kann. Insbesondere bei größeren Gewebeteilen sind Mehrfachschnitte notwendig, die zu Ausfransungen und stehenbleibenden Gewebefetzen führen.

Somit besteht der Nachteil des bekannten Instruments darin, dass das abzutrennende Gewebe nicht sehr effizient und einwandfrei abgetrennt werden kann.

Es ist daher Aufgabe der vorliegenden Erfindung ein Instrument der eingangs genannten Art dahingehend weiterzubilden, dass die Schneidleistung bzw. Schneidwirkung verbessert wird.

Erfindungsgemäß wird diese Aufgabe dadurch gelöst, dass die Öffnungen des Schneidelements, in einer Umfangsrichtung gesehen, unterschiedlich breit sind.

Diese Maßnahme hat unter anderem den erheblichen Vorteil, dass dadurch, dass die Öffnungen des Schneidelements, in einer Umfangsrichtung gesehen, unterschiedlich breit sind, unterschiedlich große Gewebestücken eingesaugt werden können.

In die Öffnungen des rotierenden Schneidelements, die in einer Umfangsrichtung gesehen, unterschiedlich breit sind, können während einer vollen Umdrehung des Schneidelements unterschiedlich große Gewebestückchen gesaugt werden und damit unterschiedlich große Gewebestückchen effizient abgetrennt werden. Es stehen also bei einer Umdrehung mehrere unterschiedliche Öffnungsbreiten zur Verfügung, um entsprechend dafür geeignet große Gewebestücke aufzunehmen.

Zusätzlich wird das abgetrennte Gewebe kleingeschnitten, wodurch ein Verstopfen des Schneidelements verhindert wird. Dies führt ebenfalls zur Erhöhung der Schneidleistung bzw. Schneidwirkung.

In einer weiteren Ausgestaltung der Erfindung ist jede der Öffnungen, in einer Umfangsrichtung gesehen, unterschiedlich breit.

Diese Maßnahme hat den Vorteil, dass während einer vollen Umdrehung des Schneidelements mehrere Schneidvorgänge erfolgen können, bei den unterschiedlich großen Gewebestückchen angesaugt und abgetrennt werden können. Somit wird eine sehr gute Effizienz der Schneidleistung und der Schneidwirkung erzielt.

In einer weiteren Ausgestaltung der Erfindung weist das Schneidelement drei Öffnungen auf.

Diese Maßnahme hat den Vorteil, dass bei dieser Zahl der Öffnungen die Möglichkeit besteht, ein Schneidelement herzustellen, bei dem die Breite der Öffnungen des Schneidelements, in einer Umfangsrichtung gesehen, sich deutlich unterscheidet. Dadurch wird erzielt, dass während einer vollen Umdrehung des Schneidelements drei Schneidvorgänge erfolgen können, bei denen Gewebestückchen, die unterschiedlich groß sind, effektiv abgetrennt werden können.

In einer weiteren Ausgestaltung der Erfindung, ist das zumindest eine Fenster des Außenschafts, in einer Umfangsrichtung gesehen, breiter als die größte Öffnung des Schneidelements.

Diese Maßnahme hat den Vorteil, dass das zumindest eine Fenster des Außenschafts während einer vollen Umdrehung des Schneidelements nie vollständig geschlossen wird.

Dadurch wird die Saugwirkung durch das Fenster hindurch nicht unterbrochen, so dass das abgetrennte Gewebe andauernd abgesaugt werden kann.

Während zuvor eingesaugtes Gewebe durch das Schneidelement abgetrennt wird, wird bereits gleichzeitig weiteres abzutrennendes Gewebe über das Fenster in eine andere Öffnung des Schneidelements eingesaugt und kann im nächsten Schneidvorgang abgetrennt werden. Dadurch kann das Gewebe effizient abgetrennt werden.

In einer weiteren Ausgestaltung der Erfindung sind die Öffnungen jeweils durch einen Steg getrennt.

Diese Maßnahme hat den Vorteil, dass durch derartige Ausgestaltungen einfach ein Schneidelement mit umfänglich versetzten Öffnungen hergestellt werden kann. Ein derartig ausgebildetes Schneidelement weist eine besonders hohe Stabilität auf.

In einer weiteren Ausgestaltung der Erfindung weisen die Stege, in einer Umfangsrichtung gesehen, eine geringere Breite auf als das zumindest eine Fenster des Außenschafts.

Diese Maßnahme hat den Vorteil, dass die Saugwirkung durch das Fenster des Außenschaftes während des gesamten Arbeitszyklus des Schneidelements, ununterbrochen ist. Durch die permanente Ansaugung des abzutrennenden Gewebes in den Außenschaft in Verbindung mit mehreren Schneidvorgängen während einer vollen Umdrehung des Schneidelements wird eine hohe Schneidleistung des erfindungsgemäßen Instruments erzielt.

In einer weiteren Ausgestaltung der Erfindung weist der Außenschaft ein einziges Fenster auf.

Diese Maßnahme hat den Vorteil, dass ein derartig ausgebildeter Außenschaft besonders gezielt seitlich an ein abzutrennendes Gewebe herangeführt werden kann.

In einer weiteren Ausgestaltung der Erfindung weist der Außenschaft mehrere umfänglich versetzt angeordnete Fenster auf.

Diese Maßnahme hat den Vorteil, dass die Schneidleistung des erfindungsgemäßen Instruments weiter verbessert werden kann. Ein Außenschaft mit mehreren Fenstern kann mit einem Schneidelement mit mehreren Öffnungen kombiniert werden, um die Schneidleistung des erfindungsgemäßen Instruments noch weiter zu erhöhen. Dadurch wird erzielt, dass wesentlich mehr Schneiden im Einsatz sind und dadurch auch mehr Gewebe in kurzer Zeit abgetrennt werden kann.

In einer weiteren Ausgestaltung der Erfindung weisen die Fenster des Außenschafts eine gleiche Geometrie auf, wie die Öffnungen des in diesem Außenschaft aufgenommenen Schneidelements.

Diese Maßnahme hat den Vorteil, dass die Schneidleistung noch weiter verbessert wird. Durch eine derartige Kombinationen arbeiten die Schneiden noch effizienter, wodurch mehr Gewebe schneller abgetrennt werden kann.

Durch eine derartige Ausgestaltung des erfindungsgemäßen Instruments wird auch ein Verstopfen des Schneidelements verhindert, da abgetrennte oder erfasste Gewebeteile in mehrere kleine Stücke aufgetrennt werden.

Es versteht sich, dass die vorstehend genannten und die nachstehend noch zu erläuternden Merkmale nicht nur in den angegebenen Kombinationen, sondern auch in anderen Kombinationen einsetzbar sind ohne den Rahmen der vorliegenden Erfindung zu verlassen.

Die Erfindung wird nachfolgend anhand eines ausgewählten Ausführungsbeispiels in Zusammenhang mit den Zeichnungen näher beschrieben und erläutert. Es zeigen:
- Fig. 1: ein medizinisches Instrument zum Schneiden von Gewebe in teilweise geschnittener Seitenansicht,
- Fig. 2: eine vergrößerte Darstellung des distalen Endabschnittes des Schneidelements von Fig. 1,
- Fig. 3: eine vergrößerte Darstellung des distalen Endes des Außenschafts von Fig. 1,
- Fig. 4: ein weiteres Ausführungsbeispiel eines Schneidelements in einer der Fig. 2 entsprechenden Darstellung,
- Fig. 5: das Ausführungsbeispiel des Schneidelements von Fig. 4, wobei das Schneidelement in einen Außenschaft hineingeschoben wird, dessen Fenster eine gleiche Geometrie aufweisen, wie die Öffnungen dieses Schneidelements,
- Fig. 6: einen Schnitt entlang der Linie VI-VI in Fig. 1,
- Fig. 7: einen der Fig. 6 entsprechenden Schnitt, der eine gegenüber Fig. 6 veränderte Drehstellung des Schneidelements zeigt, und
- Fig. 8: einen der Fig. 6 entsprechenden Schnitt, der eine gegenüber Fig. 7 veränderte Drehstellung des Schneidelements zeigt.

Ein in den Figuren dargestelltes medizinisches Instrument zum Schneiden von Gewebe ist in seiner Gesamtheit mit der Bezugsziffer 10 bezeichnet.

Das medizinische Instrument 10 weist einen rohrförmigen Außenschaft 12 auf, der an seinem proximalen Ende 13 mit einem Gehäuse 16 verbunden ist.

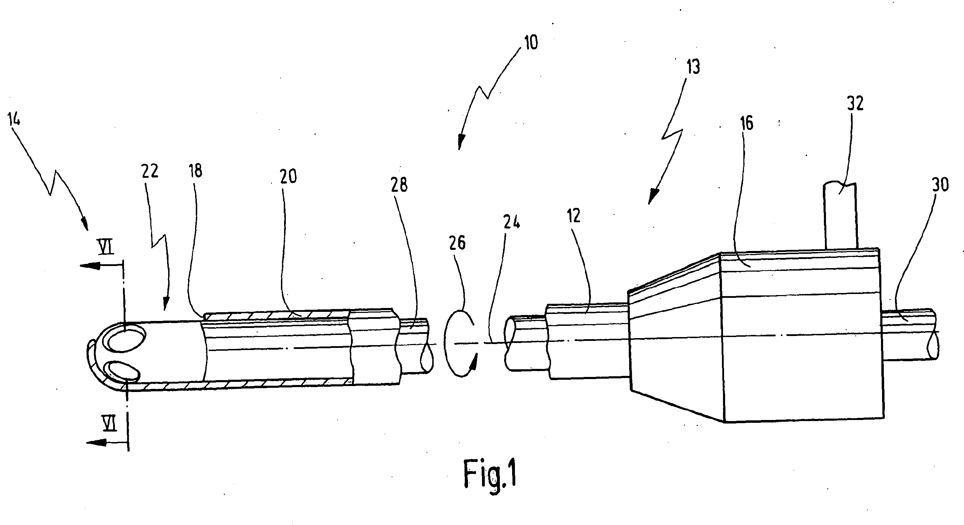

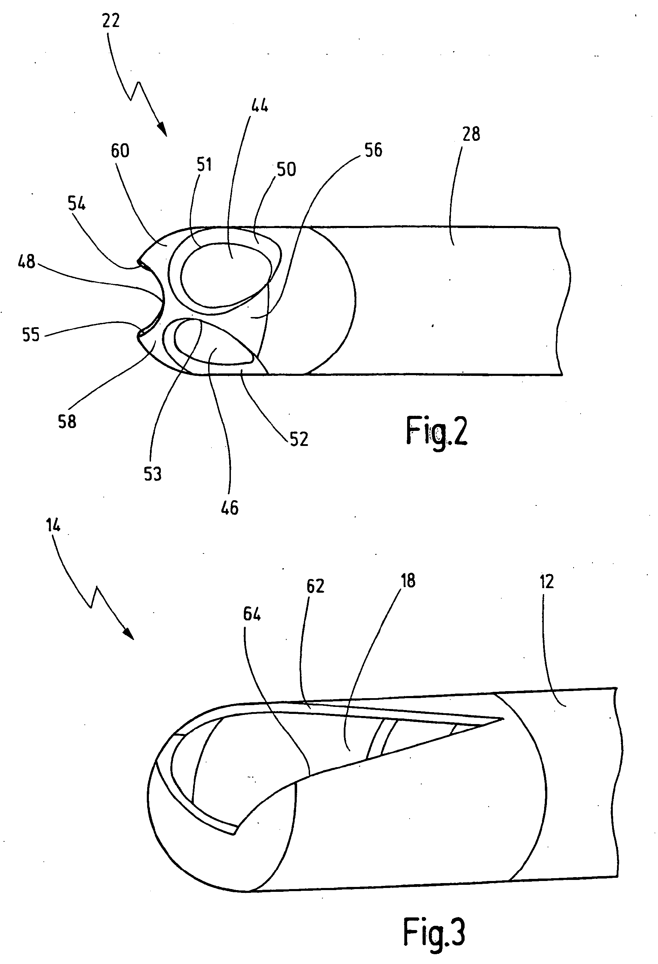

In seinem abgerundeten und geschlossenen distalen Ende 14 weist der Außenschaft 12 ein Fenster 18 auf. Das Fenster 18 ist dadurch gebildet, dass in einer Wand 20 des Außenschaftes 12 eine umfänglich und axial begrenzte etwa dreieckförmige Öffnung ausgebildet ist, wie aus Fig. 1 in Verbindung mit Fig. 3 hervorgeht.

Im Bereich des Fensters 18 ist in dem Außenschaft 12 ein Schneidelement 22 angeordnet. Das Schneidelement 22 ist in dem Außenschaft 12 um eine Längsachse 24 des medizinischen Instruments 10 rotierbar aufgenommen und kann entsprechend einem Pfeil 26 in dem ruhenden Außenschaft 12 in Rotation versetzt werden.

Das Schneidelement 22 ist an dem distalen Ende 14 eines rohrförmigen Innenschafts 28 einstückig mit diesem verbunden angeordnet, der an dem proximalen Ende 13 mit einer Antriebswelle 30 verbunden ist. Die Antriebswelle 30 wird über einen nichtdargestellten Motor in Rotation gemäß dem Pfeil 26 versetzt, wobei die Drehbewegung der Antriebswelle 30 über den Innenschaft 28 auf das damit einstückig verbundene Schneidelement 22 übertragen wird.

Ferner ist das Instrument 10 mit einer nicht dargestellten Saugquelle verbindbar, deren Saugleitung an einem Stutzen 32 des Gehäuses 16 anschließbar ist. Bei angeschlossener und eingeschalteter Saugquelle bildet sich ein Saugstrom durch den Innenschaft 28 um das Schneidelement 22 bis zum Fenster 18 auf, der von dem Fenster 18 zum Stutzen 32 gerichtet ist.

Wie aus der vergrößerten Darstellung von Fig. 2 ersichtlicht ist, weist das Schneidelement 22 drei umfänglich angeordnete Öffnungen 44, 46, 48 auf, die, in einer Umfangsrichtung gesehen, unterschiedlich breit sind. Die größte Breite weist die Öffnung 44 auf, die kleinste dagegen die Öffnung 48.

Die Öffnungen 44, 46, 48 sind durch drei Stege 56, 58, 60 voneinander getrennt.

Die Öffnungen 44, 46, 48 weisen jeweils einen Rand 50, 52, 54 auf. Jeder Rand 50, 52, 54 ist als Schneide 51, 53, 55 ausgebildet.

In Fig. 3 ist eine vergrößerte Darstellung des distalen Endes 14 des Außenschafts 12 von Fig. 1 dargestellt.

Das an dem distalen Ende 14 des Außenschaftes 12 angeordnete Fenster 18 weist einen Rand 62 auf, der als Schneide 64 ausgebildet ist.

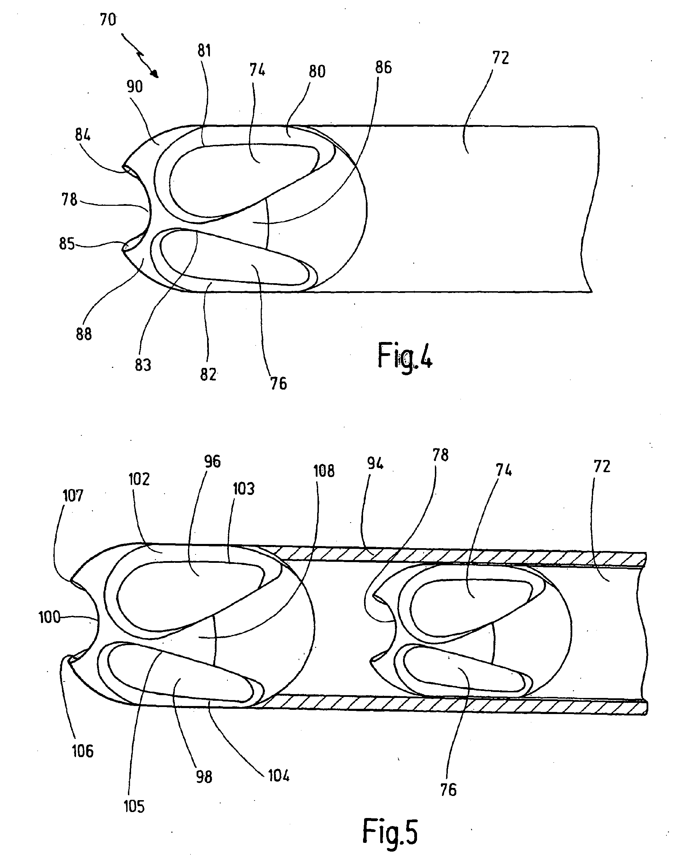

In Fig. 4 ist ein weiteres Ausführungsbeispiel eines Schneidelements 70 dargestellt, das sich von dem in Fig. 2 dargestellten Ausführungsbeispiel hinsichtlich der Länge der drei Öffnungen unterscheidet.

Bei dem in Fig. 4 dargestellten Ausführungsbeispiel weist das Schneidelement 70 ebenfalls drei Öffnungen 74, 76, 78, die umfänglich versetzt angeordnet sind und durch drei Stege 86, 88, 90 voneinander getrennt sind. Die Öffnungen 74, 76, 78 weisen ebenfalls jeweils einen als Schneide 81, 83, 85 ausgebildeten Rand 80, 82, 84 auf.

Im Vergleich zu dem Ausführungsbeispiel von Fig. 2 ist zu erkennen, dass die axiale Länge der Öffnungen 74, 76, 78 größer gegenüber der Länge der Öffnungen 44, 46, 48 ist.

In Fig. 5 ist eine Situation dargestellt, in der das Schneidelement 70 von Fig. 4 in einen Außenschaft 94 hineingeschoben wird, dessen distales Ende 92 drei Fenster 96, 98, 100 aufweist, die die gleiche Geometrie und Form aufweisen, wie die drei Öffnungen 74, 76, 78 des Schneidelements 70.

Die umfänglich angeordneten Fenster 96, 98, 100 sind durch drei Stege 108, 110, 112 voneinander getrennt.

Die Fenster 96, 98, 100 weisen ebenfalls jeweils einen als Schneide 103, 105, 107 ausgebildeten Rand 102, 104, 106 auf.

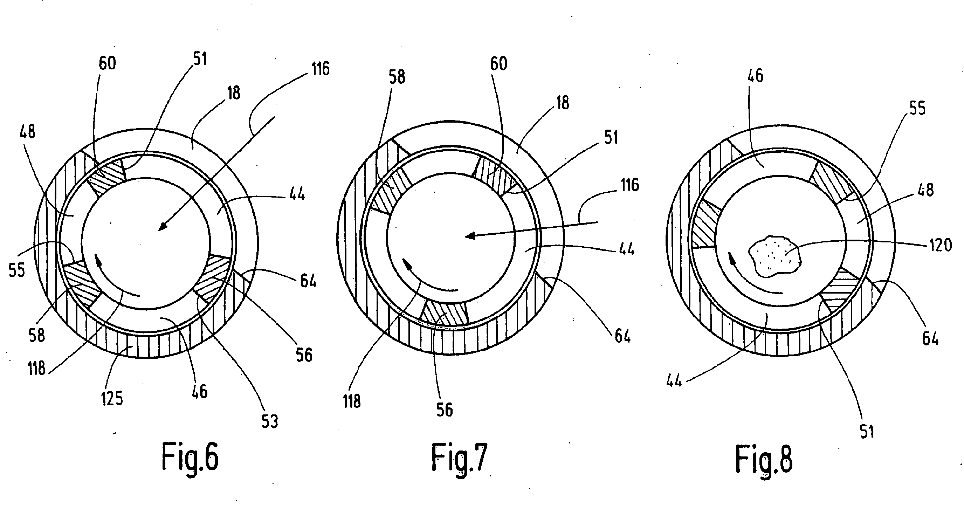

Der Einsatz des medizinischen Instruments 10 von Fig. 1 soll im Ablauf der Figuren 6 bis 8 kurz erläutert werden.

Aus Fig. 6 sind drei Öffnungen 44, 46, 48 des Schneidelements 22 ersichtlich, die durch drei Stege 56, 58, 60 voneinander getrennt sind.

Die Schneiden 51, 53, 55 wirken beim Umlaufen des Schneidelements 22 in dem Außenschaft 12 in Umlaufrichtung, die durch den Pfeil 118 angezeigt ist, mit der Schneide 64, die an dem seitlichen Rand 62 des Fensters 18 des Außenschafts 12 ausgebildet ist, schneidend zusammen.

Zum Schneiden bzw. Abtrennen von Gewebe, das durch den Pfeil 116 angezeigt ist, wird das Instrument 10 im Bereich des Fensters 18 an das Gewebe angelegt, das Schneidelement 22 wird in Rotation versetzt und mittels der Saugquelle ein Saugstrom durch den Innenschaft 28 bis zu dem Fenster 18 eingestellt. Durch die im Bereich des Fensters 18 bestehende Saugwirkung wird das abzutrennende Gewebe durch das Fenster 18 und die Öffnung 44 eingesaugt. Durch das Vorbeilaufen der Schneide 51 des Schneidelements 22 an der Schneide 64 des Fensters 18, wie es aus Fig. 7 und 8 ersichtlich, wird das eingesaugte abzutrennende Gewebe abgetrennt. Das abgetrennte Gewebe 120 wird durch den Innenschaft 28 zum proximalen Ende 13 des Instruments 10 hin abgesaugt.

Wie aus den Figuren 6 bis 8 hervorgeht, weist jeder der Öffnungen 44, 46, 48 eine geringere Breite auf als das Fenster 18 des Außenschafts 12.

Die Stege 56, 58, 60 weisen ebenfalls eine geringere Breite auf als das Fenster 18 des Außenschafts 12.

Dadurch bleibt das Fenster 18 des Außenschafts 12 stets zumindest teilweise offen, so dass die im Bereich des Fensters 18 bestehende Saugwirkung in keiner Drehstellung des Schneidelements 22 unterbrochen ist.

Bei einer Kombination von dem Schneidelement 22 mit dem Außenschaft 12, der nur ein einziges Fenster 18 aufweist, können während eines voller Umdrehung des Schneidelements 22 drei Schneidvorgänge stattfinden, wobei jeweils Gewebestücke passender Größe abgetrennt werden.

Durch Kombinieren des Schneidelements 70 mit dem Außenschaft 92, der drei Fenster 96, 98, 100 aufweist, wird eine noch höhere Schneidleistung erzielt, insbesondere, wenn Gewebe rundum abgetrennt werden soll.

## Patentansprüche

1. Medizinisches Instrument zum Schneiden von Gewebe, mit einem rohrförmigen Außenschaft (12, 94), der im Bereich seines distalen Endes (14, 92) zumindest ein Fenster (18; 96, 98, 100) mit zumindest einer Schneide (64; 103, 105, 107) aufweist, und mit einem rohrförmigen, um eine Längsachse (24) rotierbaren, in dem Außenschaft (12, 94) aufgenommenen Innenschaft (28, 72), der an seinem distalen Ende (14, 92) ein im Bereich des zumindest einen Fensters (18; 96, 98, 100) des Außenschafts (12, 94) angeordnetes, mit mehreren umfänglich angeordneten Öffnungen (44, 46, 48; 74, 76, 78) versehenes Schneidelement (22, 70) aufweist, wobei jede der Öffnungen (44, 46, 48; 74, 76, 78) des Schneidelements (22, 70) zumindest eine Schneide (51, 53, 55; 81, 83, 85) aufweist, die bei in Rotation versetztem Schneidelement (22, 70), mit der zumindest einen Schneide (64; 103, 105, 107) des Außenschafts (12, 94) schneidend zusammenwirkt, **dadurch gekennzeichnet, dass** die Öffnungen (44, 46, 48; 74, 76, 78) des Schneidelements (22, 70), in einer Umfangsrichtung gesehen, unterschiedlich breit sind.

2. Medizinisches Instrument nach Anspruch 1, **dadurch gekennzeichnet, dass** jede der Öffnungen (44, 46, 48; 74, 76, 78), in einer Umfangsrichtung gesehen, unterschiedlich breit ist.

3. Medizinisches Instrument nach Anspruch 1 oder 2, **dadurch gekennzeichnet, dass** das Schneidelement (22, 70) drei Öffnungen (44, 46, 48; 74; 76, 78) aufweist.

4. Medizinisches Instrument nach einem der Ansprüche 1 bis 3, **dadurch gekennzeichnet, dass** das zumindest eine Fenster (18; 96, 98, 100) des Außenschafts (12, 94), in einer Umfangsrichtung gesehen, breiter ist als die größte Öffnung (44, 74) des Schneidelements (22, 70).

5. Medizinisches Instrument nach einem der Ansprüche 1 bis 4, **dadurch gekennzeichnet, dass** die Öffnungen (44, 46, 48; 74, 76, 78) jeweils durch einen Steg (56, 58, 60; 108, 110, 112) getrennt sind.

6. Medizinisches Instrument nach Anspruch 5, **dadurch gekennzeichnet, dass** die Stege (56, 58, 60; 108, 110, 112) in einer Umfangsrichtung gesehen, eine geringere Breite aufweisen als das zumindest eine Fenster (18; 96, 98, 100) des Außenschafts (12, 94).

7. Medizinisches Instrument nach einem der Ansprüche 1 bis 6, **dadurch gekennzeichnet, dass** der Außenschaft (12) ein einziges Fenster (18) aufweist.

8. Medizinisches Instrument nach einem der Ansprüche 1 bis 7, **dadurch gekennzeichnet, dass** der Außenschaft (94) mehrere umfänglich versetzt angeordnete Fenster (96, 98, 100) aufweist.

9. Medizinisches Instrument nach einem der Ansprüche 1 bis 8, **dadurch gekennzeichnet, dass** die Fenster (96, 98, 100) des Außenschafts (94) eine gleiche Geometrie aufweisen, wie die Öffnungen (74, 76, 78) eines in diesem Außenschaft (94) aufgenommenen Schneidelements (70).

## Claims

1. Medical instrument for cutting tissue, with a tubular outer shaft (12, 94) which, in the area of its distal end (14, 92), has at least one window (18; 96, 98, 100) with at least one blade (64; 103, 105, 107), and with a tubular inner shaft (28, 72) which is rotatable about a longitudinal axis (24), is received in the outer shaft (12, 94) and has, at its distal end (14, 92), a cutting element (22, 70) which is arranged in the area of the at least one window (18; 96, 98, 100) of the outer shaft (12, 94) and is provided with several circumferentially arranged openings (44, 46, 48; 74, 76, 78), each of the openings (44, 46, 48; 74, 76, 78) of the cutting element (22, 70) having at least one blade (51, 53, 55; 81, 83, 85) which, when the cutting element (22, 70) is moved in rotation, cooperates in a cutting action with the at least one blade (64; 103, 105, 107) of the outer shaft (12, 94), **characterized in that** the openings (44, 46, 48; 74, 76, 78) of the cutting element (22, 70) have different widths, seen in a circumferential direction.

2. Medical instrument of claim 1, **characterized in that** each of the openings (44, 46, 48; 74, 76, 78) have a different width seen in a circumferential direction.

3. Medical instrument of claim 1 or 2, **characterized in that** the cutting element (22, 70) has three openings (44, 46, 48; 74, 76, 78).

4. Medical instrument of anyone of claims 1 through 3, **characterized in that** the at least one window (18; 96, 98, 100) of the outer shaft (12, 94), seen in a circumferential direction, is wider than a largest opening (44, 74) of the cutting element (22, 70).

5. Medical instrument of anyone of claims 1 through 4, **characterized in that** the openings (44, 46, 48; 74, 76, 78) are each separated by a web (56, 58, 60; 108, 110, 112).

6. Medical instrument of claim 5, **characterized in that** the webs (56, 58, 60; 108, 110, 112), seen in a circumferential direction, have a smaller width than said at least one window (18; 96, 98, 100) of the outer shaft (12, 94).

7. Medical instrument of anyone of claims 1 through 6, **characterized in that** the outer shaft (12) has a single window (18).

8. Medical instrument of anyone of claims 1 through 7, **characterized in that** the outer shaft (94) has several windows (96, 98, 100) which are circumferentially offset one to another.

9. Medical instrument of anyone of claims 1 through 8, **characterized in that** the windows (96, 98, 100) of the outer shaft (94) have a same geometry as the openings (74, 76, 78) of a cutting element (70) received in this outer shaft (94).

## Revendications

1. Instrument médical destiné à couper des tissus, avec un corps extérieur tubulaire (12, 94) qui présente, dans la région de son extrémité distale (14, 92), au moins une fenêtre (18 ; 96, 98, 100) pourvue d'au moins une arête coupante (64 ; 103, 105, 107), et avec un corps intérieur tubulaire (28, 72), qui est rotatif autour d'un axe longitudinal (24) et reçu dans le corps extérieur (12, 94), et qui présente à son extrémité distale (14, 92) un élément coupant (22, 70) disposé dans la région de la fenêtre au moins unique (18 ; 96, 98, 100) du corps extérieur (12, 94) et pourvu de plusieurs ouvertures circonférentiellement disposées (44, 46, 48 ; 74, 76, 78), sachant que chacune des ouvertures (44, 46, 48 ; 74, 76, 78) de l'élément coupant (22, 70) présente au moins une arête coupante (51, 53, 55 ; 81, 83, 85) qui, lorsque l'élément coupant (22, 70) est mis en rotation, coopère en une action de coupe avec l'arête coupante au moins unique (64 ; 103, 105, 107) du corps extérieur (12, 94), **caractérisé en ce que** les ouvertures (44, 46, 48 ; 74, 76, 78) de l'élément coupant (22, 70) sont de différentes largeurs, considérées dans une direction circonférentielle.

2. Instrument médical selon la revendication 1, **caractérisé en ce que** chacune des ouvertures (44, 46, 48 ; 74, 76, 78) est d'une largeur différente, considérée dans une direction circonférentielle.

3. Instrument médical selon la revendication 1 ou 2, **caractérisé en ce que** l'élément coupant (22, 70) présente trois ouvertures (44, 46, 48 ; 74, 76, 78).

4. Instrument médical selon l'une des revendications 1 à 3, **caractérisé en ce que** la fenêtre au moins unique (18 ; 96, 98, 100) du corps extérieur (12, 94) est, considérée dans une direction circonférentielle, plus large que la plus grande ouverture (44, 74) de l'élément coupant (22, 70).

5. Instrument médical selon l'une des revendications 1 à 4, **caractérisé en ce que** les ouvertures (44, 46, 48 ; 74, 76, 78) sont respectivement séparées par une nervure (56, 58, 60 ; 108, 110, 112).

6. Instrument médical selon la revendication 5, **caractérisé en ce que** les nervures (56, 58, 60 ; 108, 110, 112) présentent, considérées dans une direction circonférentielle, une plus petite largeur que la fenêtre au moins unique (18 ; 96, 98, 100) du corps extérieur (12, 94).

7. Instrument médical selon l'une des revendications 1 à 6, **caractérisé en ce que** le corps extérieur (12) présente une unique fenêtre (18).

8. Instrument médical selon l'une des revendications 1 à 7, **caractérisé en ce que** le corps extérieur (94) présente plusieurs fenêtres (96, 98, 100) disposées en étant circonférentiellement décalées.

9. Instrument médical selon l'une des revendications 1 à 8, **caractérisé en ce que** les fenêtres (96, 98, 100) du corps extérieur (94) présentent la même géométrie que les ouvertures (74, 76, 78) d'un élément coupant (70) reçu dans ce corps extérieur (94).
